# EUROPEAN PATENT APPLICATION

(11) **EP 4 803 608 A1**
(43) Date of publication of application: **09.09.2026**
(21) Application number: 24885621.3
(22) Date of filing: 25.10.2024
(51) Int. Cl.: C12N 1/04, C12N 5/071

(54) **CELL PRESERVATION LIQUID AND CELL PRESERVATION METHOD**

(30) Priority: 31.10.2023 JP 2023186162
(71) Applicant: Central Institute for Experimental Medicine and Life Science, Kawasaki-shi, Kanagawa 210-0821 (JP)
(72) Inventor: HIGUCHI Yuichiro, Kawasaki-shi, Kanagawa 210-0821 (JP); SUEMIZU Hiroshi, Kawasaki-shi, Kanagawa 210-0821 (JP)
(74) Representative: Büchel, Edwin
(86) International application number: PCT/JP2024/038157
(87) International publication number: WO 2025/094848

(57) **Abstract**

The present invention relates to a method of preserving adherent cells, comprising storing adherent cells in a non-frozen state under refrigeration in a cell preservation solution comprising a high-molecular-weight polymer; a method of culturing adherent cells, comprising culturing adherent cells in two dimensional culture, after preservation by the aforementioned method; and a cell preservation solution comprising a high-molecular-weight polymer suitable for use in these methods.

## Description

### Technical Field

The present invention relates to a cell preservation solution and a cell preservation method that enable adherent cells to be preserved under refrigeration for a long period of time.

### Background Art

Planar culture of primary human hepatocytes (PHH) is widely used in the development process of new drugs, for example in pharmacokinetic and toxicity analysis studies. In monolayer cultures of PHH, it is known that the expression levels of drug-metabolizing enzymes decrease as the cell density decreases (Non Patent Literature 1). To maintain the characteristics of PHH *in vitro* for a long period of time, it is important to maintain the cultured cells at as high a density as possible.

Commercially available frozen PHH are classified into two types: plateable grade (adherent cells), which can establish an adherent culture on a culture substrate, and suspension grade (suspension cells), which cannot establish an adherent culture. Plateable grade PHH can be used in adherent culture for generally four weeks or more, allowing for long-term *in vitro* culture. Therefore, plateable grade PHH are more useful than suspension grade PHH, which can only be used for approximately eight hours after thawing. However, a method for selectively producing plateable grade frozen PHH has not yet been established. It is said that only approximately 30% of commercially available frozen PHH are plateable grade.

When PHH are suspended in a preservation solution and placed under refrigeration conditions (4°C, on ice), they can be preserved for a short period of time while maintaining their high adhesion capacity onto culture substrates; however, PHH stored under refrigeration for a long period of time show a decrease in adhesion capacity onto culture substrates. When cells such as PHH are transported over long distances, prolonged storage is required; for example, transport from Japan to Europe requires at least 48 to 72 hours, preferably about 96 hours, even by air on a door-to-door basis. Therefore, there is a need for the development of a cell preservation method that enables adherent cells such as PHH to maintain their high adhesion capacity onto culture substrates even after long-term, unfrozen storage.

Non-Patent Literature 2 reports that for PHH stored under refrigeration in a plurality of types of organ preservation solutions, the viability rate and adhesion capacity onto collagen I-coated plates were maintained for up to approximately 24 hours of storage, but significantly decreased at longer storage times.

Non-Patent Literature 3 reports that storing PHH under refrigeration in a cell preservation solution with a special composition containing an iron chelator and then warming it mitigates cell death caused by cold stress. However, the cell preservation solution used in Non-Patent Literature 3 has a complex composition and is difficult to prepare.

Patent Literature 1 discloses a method for culturing vascular smooth muscle cells comprising culturing them in suspension in a medium composition comprising deacylated gellan gum or a salt thereof. However, Patent Literature 1 does not disclose the adhesion capacity of cells stored under refrigeration in the medium composition to a culture substrate, nor does it mention any effects on cells other than vascular smooth muscle cells.

Patent Literature 2 discloses that cells or tissues can be stored in a non-frozen state while maintaining good survivability for a long period of time, in a liquid composition comprising deacylated gellan gum or a salt thereof and an acidic polysaccharide such as alginic acid. However, Patent Literature 2 does not disclose the adhesion capacity of cells stored under refrigeration in the liquid composition onto a culture substrate.

### Citation List

### Patent Literature

Patent Literature 1: International Publication WO2016/121896.
Patent Literature 2: International Publication WO2019/049985.

### Non Patent Literature

Non Patent Literature 1: Yamasaki et al., PLOS ONE, 15(9): (2020) e0237809
Non Patent Literature 2: Duret et al., Cell Transplantation, Vol. 24, pp. 2541-2555 (2015)
Non Patent Literature 3: Pless et al., Cell Transplantation, Vol. 21, pp. 23-37 (2012)

### Summary of Invention

### Technical Problem

A problem underlying the present invention is to provide a cell preservation solution and a cell preservation method that enable adherent cells to be stored under refrigeration for a long period of time while maintaining their adhesion capacity onto culture substrates. A further problem underlying the present invention is to provide a cell culture method that enables two-dimensional culture of adherent cells after long-term storage under refrigeration while maintaining their cellular characteristics.

### Solution to Problem

The inventors have conducted intensive studies to solve the above-mentioned problems. As a result, the inventors found that when adherent cells were stored under refrigeration for a long period of time in a cell preservation solution comprising a high-molecular-weight polymer such as deacylated gellan gum or a salt thereof, the cells did not settle to the bottom of the container but remained in suspension, and their adhesion capacity onto culture substrates in the subsequent two-dimensional culture was significantly increased, leading to the completion of the present invention.

Specifically, the present invention includes the following aspects.
[1] A method of preserving adherent cells, comprising storing adherent cells in a non-frozen state under refrigeration in a cell preservation solution comprising a high-molecular-weight polymer.
[2] The method according to [1] above, wherein the high-molecular-weight polymer comprises deacylated gellan gum or a salt thereof.
[3] The method according to [1] or [2] above, wherein the adherent cells are stored under refrigeration while maintaining their adhesion capacity onto a culture substrate in two-dimensional culture.
[4] The method according to any one of [1] to [3] above, wherein the adherent cells are stored under refrigeration for up to 100 hours.
[5] The method according to any one of [1] to [4] above, wherein the adherent cells are stored under refrigeration for 70 hours or more.
[6] The method according to any one of [1] to [5] above, wherein the adherent cells are hepatocytes.
[7] The method according to any one of [1] to [6] above, wherein the adherent cells are stored in suspension in a cell preservation solution.
[8] The method according to any one of [1] to [7] above, wherein the cell preservation solution further comprises an iron chelator.
[9] The method according to [8] above, wherein the iron chelator comprises deferoxamine.
[10] A method of culturing adherent cells, comprising storing the adherent cells under refrigeration using the method according to any one of [1] to [9] above, and then culturing the adherent cells in two-dimensional culture.
[11] The method according to [10] above, wherein the adherent cells are seeded on a collagen-coated culture substrate and cultured in two-dimensional culture.
[12] The method according to [10] or [11] above, wherein the adherent cells that have been stored under refrigeration are warmed and then subjected to two-dimensional culture.
[13] A cell preservation solution comprising a high-molecular-weight polymer, for preserving adherent cells in a non-frozen state under refrigeration.
[14] The cell preservation solution according to [13] above, wherein the high-molecular-weight polymer comprises deacylated gellan gum or a salt thereof.
[15] The cell preservation solution according to [13] or [14] above, for preserving adherent cells under refrigeration while maintaining their adhesion capacity onto a culture substrate in two-dimensional culture.
[16] The cell preservation solution according to any one of [13] to [15] above, for preserving adherent cells under refrigeration for up to 100 hours.
[17] The cell preservation solution according to any one of [13] to [16] above, wherein the adherent cells are hepatocytes.
[18] The cell preservation solution according to any one of [13] to [17] above, which further comprises an iron chelator.

The present specification includes the contents disclosed in Japanese Patent Application No. 2023-186162 from which the present application claims the priority.

### Advantageous Effects of Invention

The present invention enables adherent cells to be stored under refrigeration for a long period of time while maintaining their adhesion capacity onto culture substrates. The present invention also enables two-dimensional culture of adherent cells after long-term storage under refrigeration while maintaining their cellular properties.

### Brief Description of Drawings

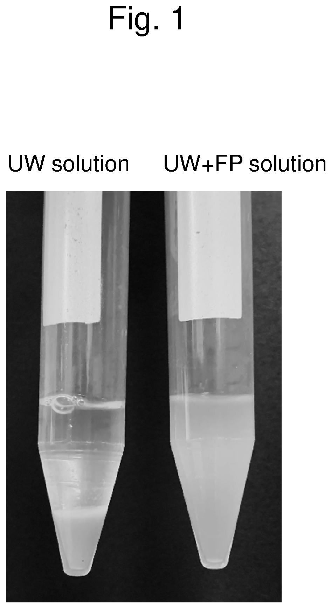

[Figure 1] Figure 1 is a photo showing the appearance of cells in preservation solution 24 hours after the start of storage under refrigeration in UW solution and UW+FP solution.

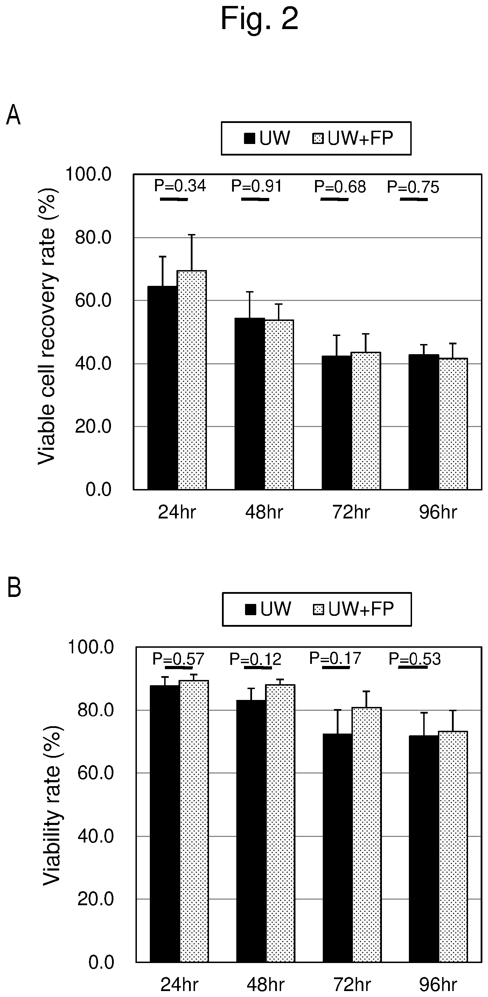

[Figure 2] Figure 2 shows the viable cell recovery rate (A) and viability rate (B) of cells stored under refrigeration in UW solution and UW+FP solution for 24, 48, 72, and 96 hours.

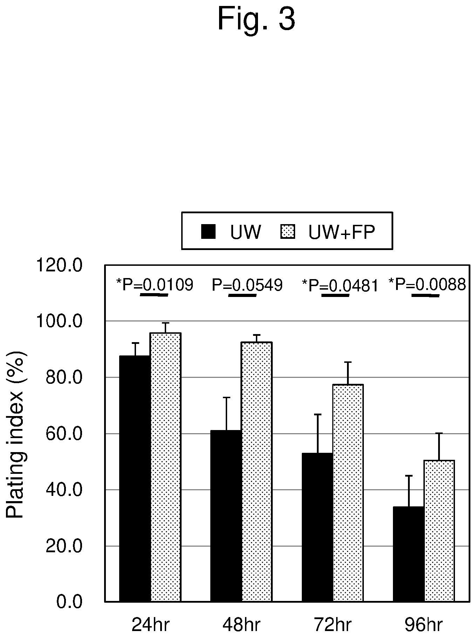

[Figure 3] Figure 3 shows the adhesion efficiency (plating index) after 24 hours of seeding on a plate of cells stored under refrigeration in UW solution or UW+FP solution for 24, 48, 72, and 96 hours.

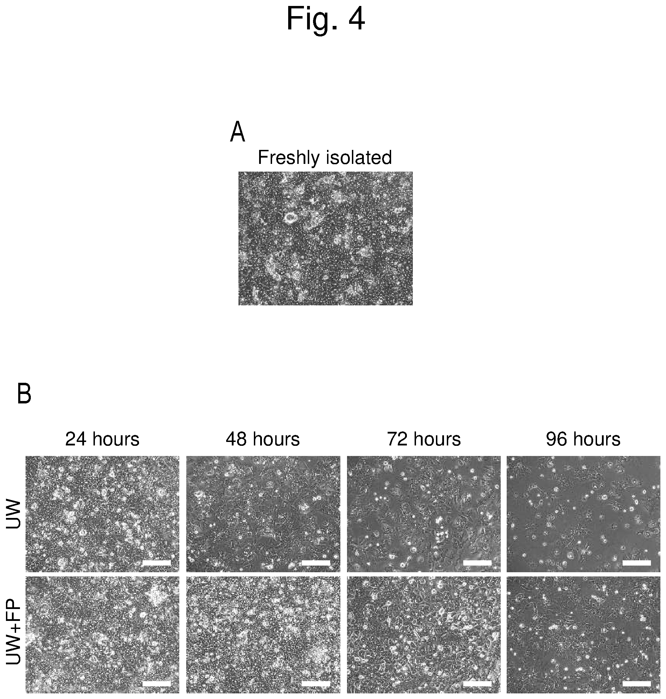

[Figure 4] Figure 4 shows phase-contrast image photos (B) after 24 hours of seeding on a plate of cells stored under refrigeration in UW solution or UW+FP solution for 24, 48, 72, or 96 hours. For comparison, a phase-contrast image (A) of freshly isolated HepaSH cells from a humanized liver is also shown. The white bar in the lower right corner of each photograph indicates a 200 µm scale.

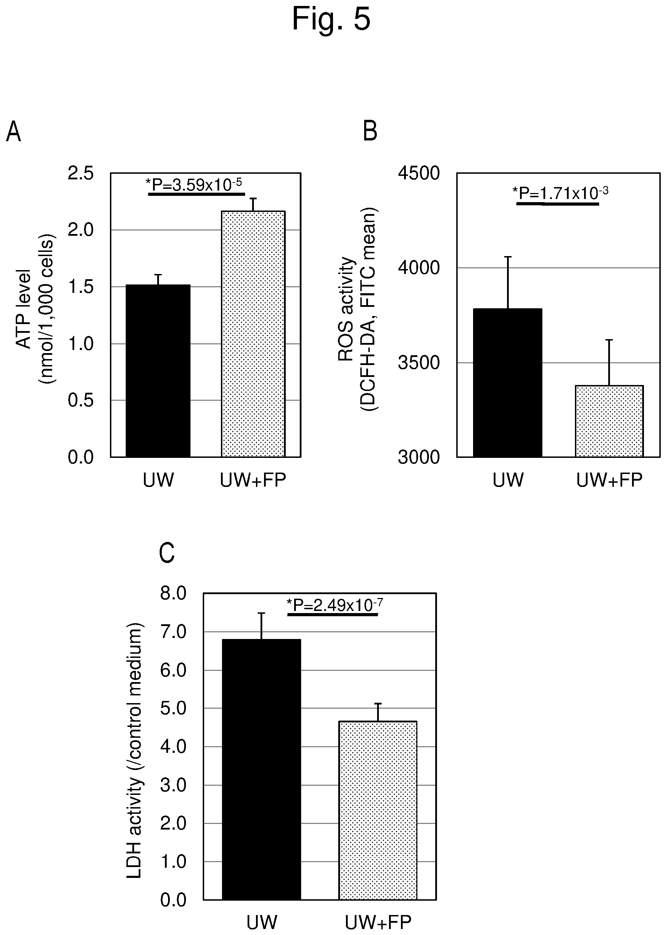

[Figure 5] Figure 5 shows the ATP level (A) in cells stored under refrigeration for 24 hours in UW solution and UW+FP solution, ROS activity (B) expressed by, as an indicator, the mean fluorescence intensity derived from DCFH-DA, and LDH activity in the medium 4 hours after seeding on a plate (C).

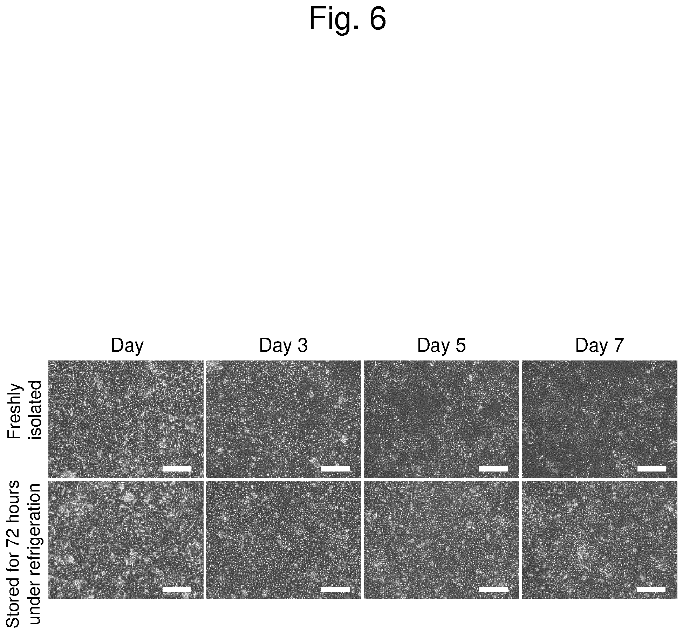

[Figure 6] Figure 6 shows phase-contrast image photos of cells seeded on a plate on Days 1, 3, 5, and 7 of maintenance culture after 72 hours of storage under refrigeration in UW+FP solution. For comparison, phase-contrast images of freshly isolated HepaSH cells from a humanized liver are also shown. The white bar in the lower right corner of each photo indicates a 200 µm scale.

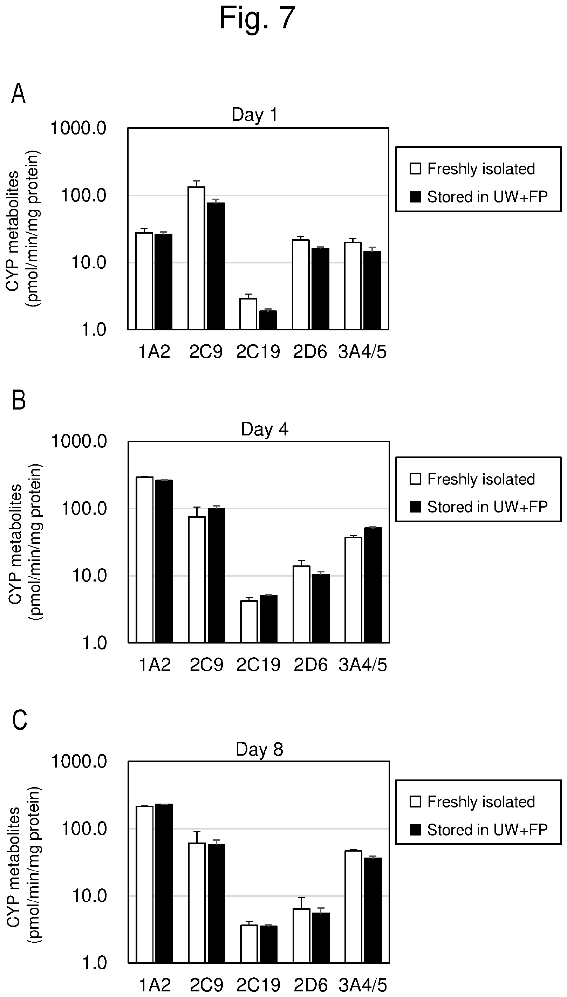

[Figure 7] Figure 7 shows drug-metabolizing enzyme activities in cells seeded on plates on Days 1 (A), 4 (B), and 8 (C) of maintenance culture after 72 hours of storage under refrigeration in UW+FP solution. 1A2, 2C9, 2C19, 2D6, and 3A4/5 in the figure denote: CYP1A2-mediated phenacetin O-deethylation activity, CYP2C9-mediated diclofenac 4'-hydroxylation activity, CYP2C19-mediated omeprazole 5'-hydroxylation activity, CYP2D6-mediated metoprolol O-demethylation activity, and CYP3A4/5-mediated midazolam 1'-hydroxylation activity, respectively.

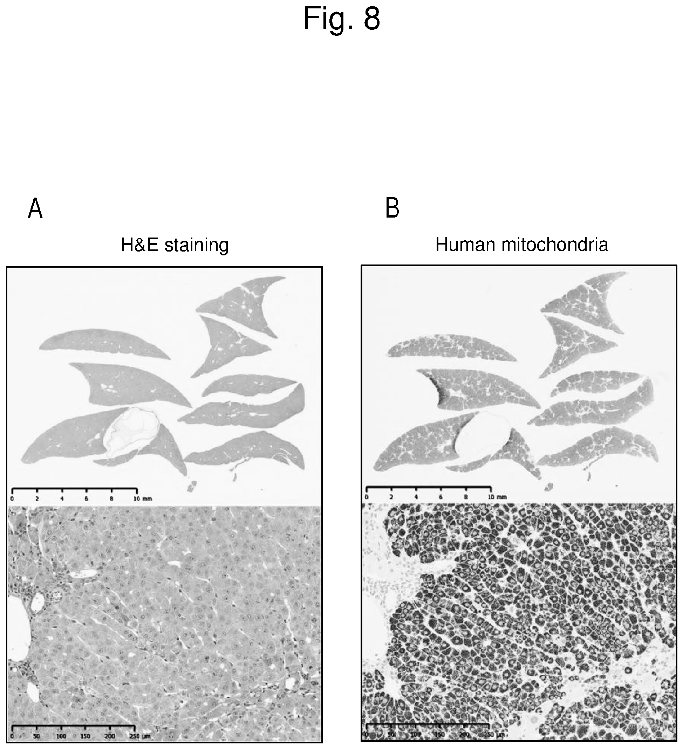

[Figure 8] Figure 8 shows photos indicating the results of immunostaining of the liver at 5 weeks after re-transplantation of HepaSH cells that had been stored under refrigeration for 72 hours in UW+FP solution and re-transplanted into the liver of a TK-NOG-hIL6 mouse after induction of liver damage. A: H&E staining, B: staining with anti-human mitochondria antibody

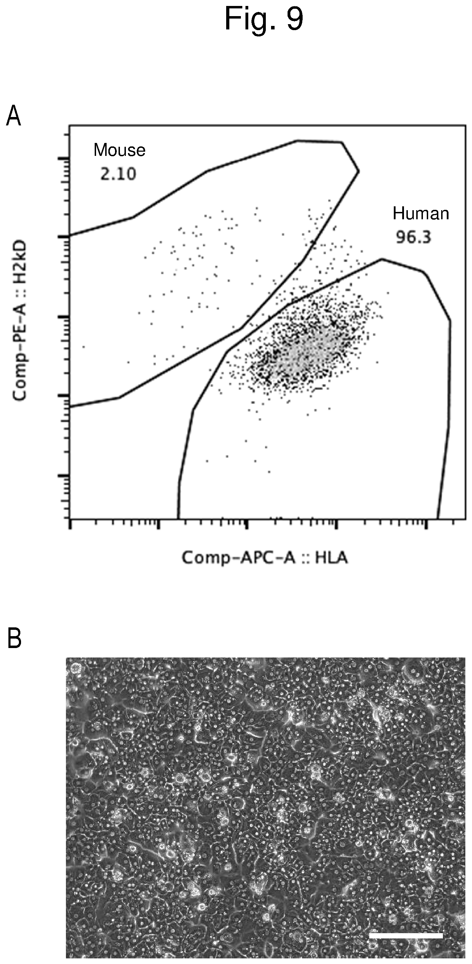

[Figure 9] Figure 9 shows the results of flow cytometry analysis (A) of cells isolated from the liver at 6 weeks after re-transplantation of HepaSH cells that had been stored under refrigeration for 72 hours in UW+FP solution and re-transplanted into the liver of a TK-NOG-hIL6 mouse after induction of liver damage. Figure 9 also shows a photo (B) showing the appearance of maintenance culture of the thus re-isolated HepaSH cells after seeding onto plates. The white bar in the lower right corner of the photo in Figure 9B indicates a 200 µm scale.

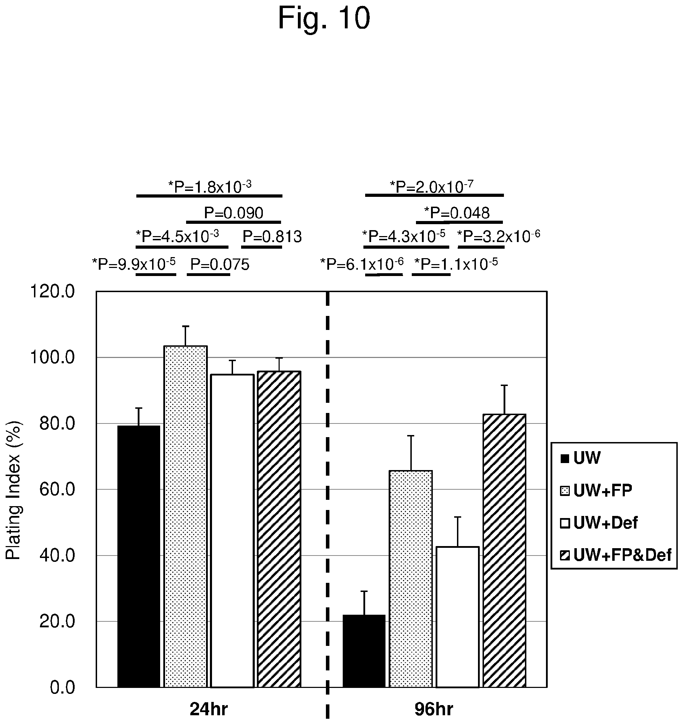

[Figure 10] Figure 10 shows adhesion efficiency after 24 hours of seeding onto plates of cells that had been stored under refrigeration for 24 or 96 hours in UW solution, UW+FP solution, UW+Def solution, or UW+FP&Def solution.

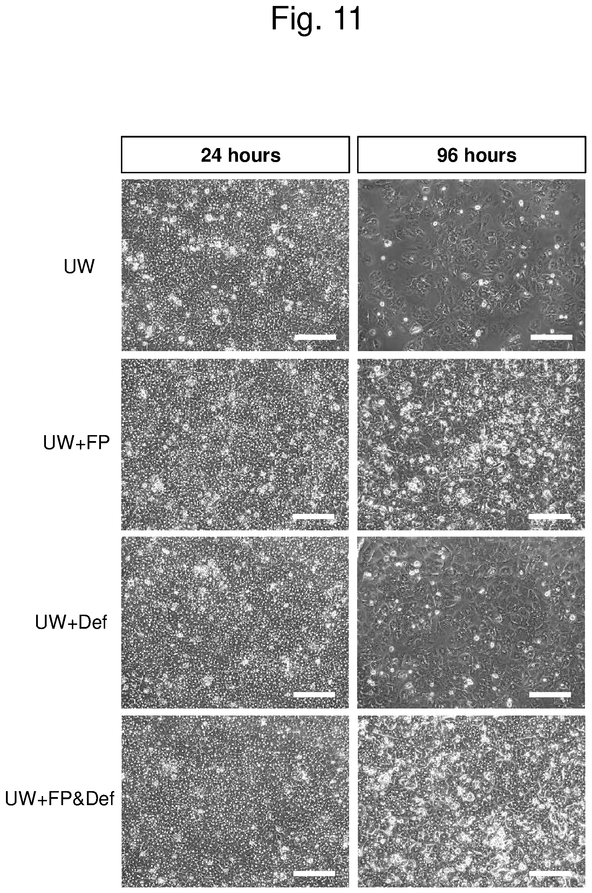

[Figure 11] Figure 11 shows phase-contrast image photos after 24 hours of seeding onto plates of cells that had been stored under refrigeration for 24 or 96 hours in UW solution, UW+FP solution, UW+Def solution, or UW+FP&Def solution. The white bar in the lower right corner of each photo indicates a 200 µm scale.

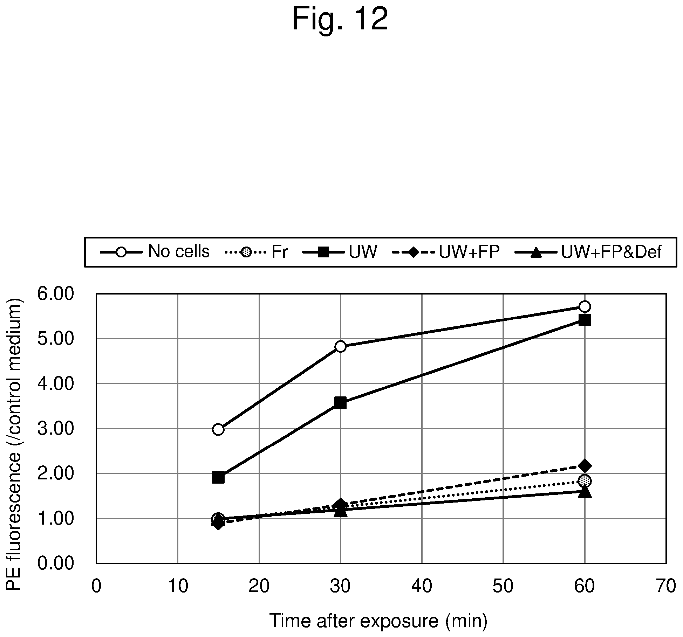

[Figure 12] Figure 12 shows the results of a permeation test of a fluorescent substance added to the medium after maintenance culture on the Transwell for 7 days of cells that had been stored under refrigeration for 96 hours in UW solution, UW+FP solution, or UW+FP&Def solution.

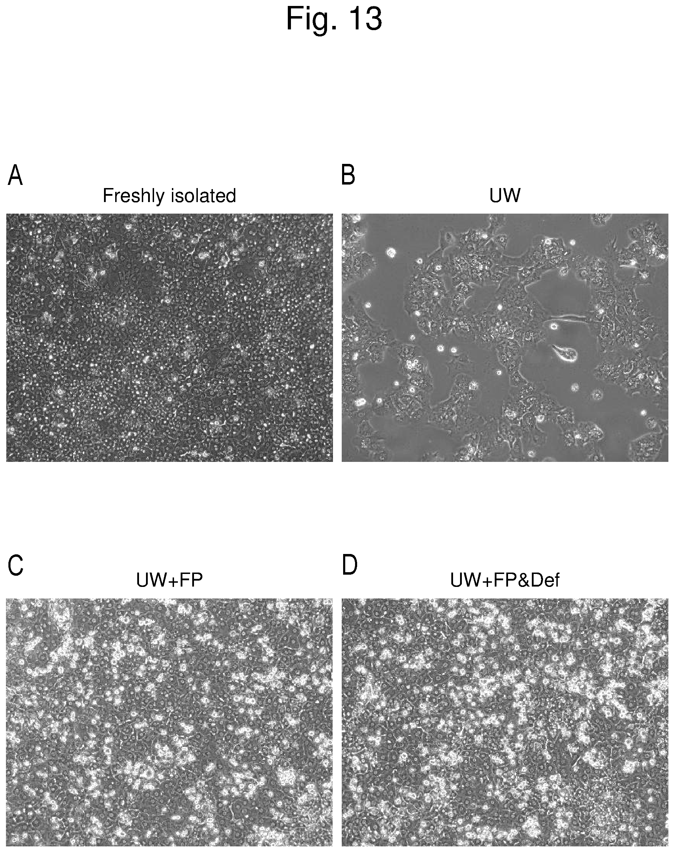

[Figure 13] Figure 13 shows phase-contrast image photos of monolayer culture at Day 7 of cells that had been stored under refrigeration for 96 hours in UW solution (B), UW+FP solution (C), or UW+FP&Def solution (D). For comparison, a phase-contrast image (A) of freshly isolated HepaSH cells from a humanized liver is also shown.

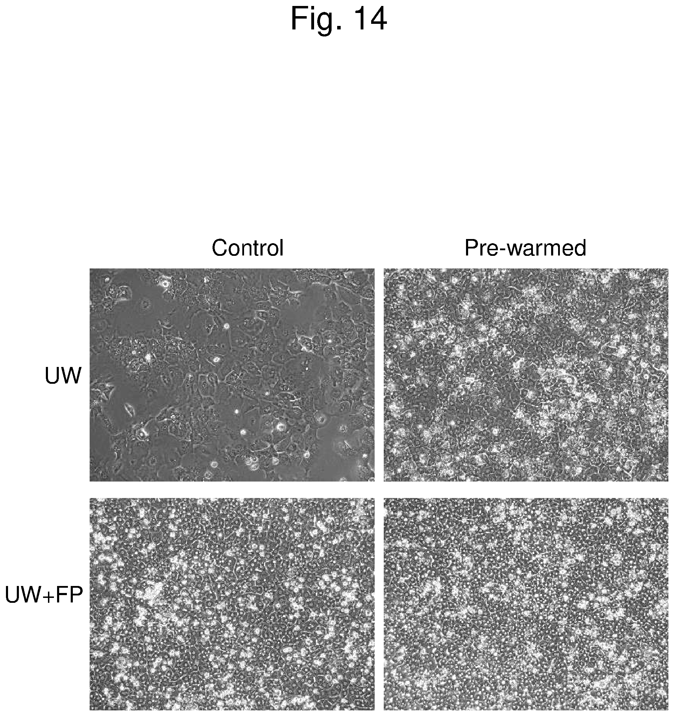

[Figure 14] Figure 14 shows phase-contrast image photos taken 24 hours after seeding cells onto collagen I-coated 24-well plates, which had been stored under refrigeration for 72 hours in UW solution or UW+FP solution, and then subjected to pre-warming treatment or not subjected to pre-warming treatment (control).

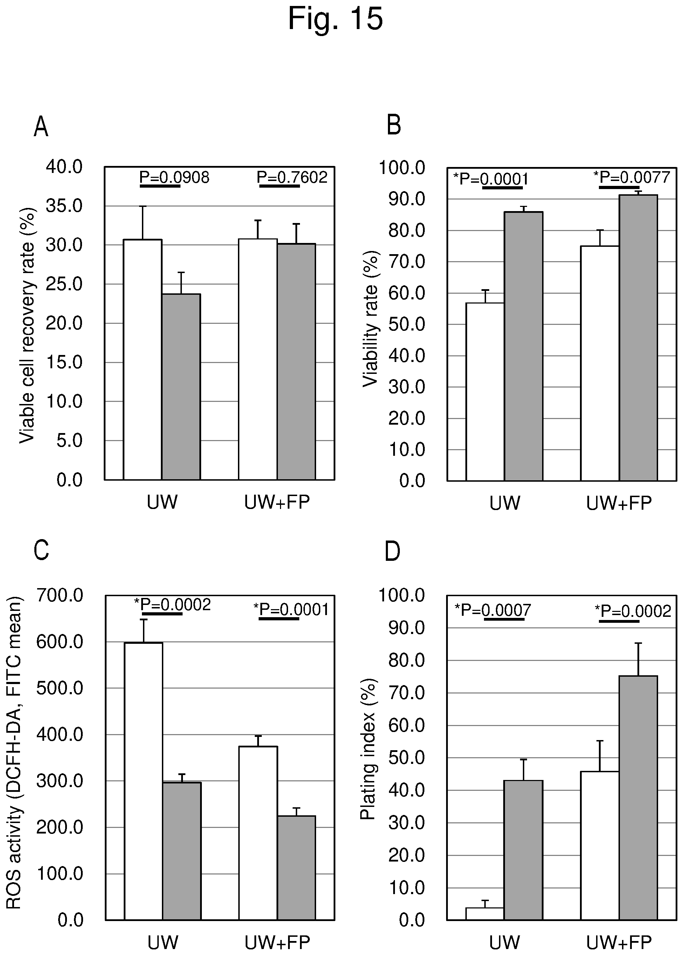

[Figure 15] Figure 15 shows the viable cell recovery rates (A), viability rates (B), ROS activities (C), and adhesion efficiency (D) of cells that were stored under refrigeration for 72 hours in UW solution or UW+FP solution and then subjected or not subjected to pre-warming treatment. Open bars: no pre-warming, shaded bars: pre-warming.

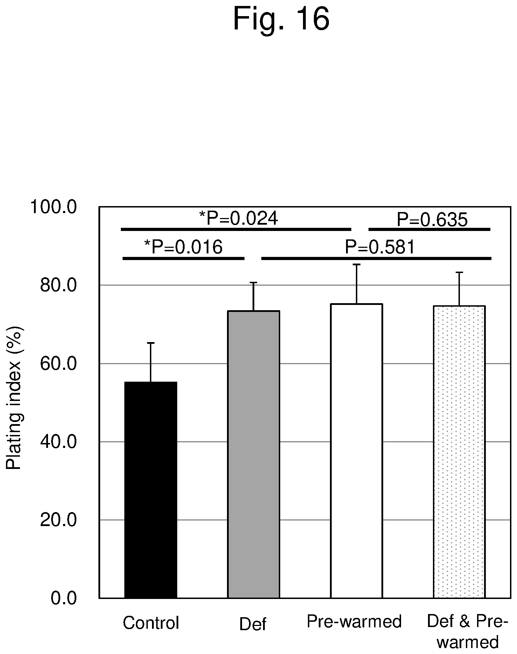

[Figure 16] Figure 16 shows adhesion efficiency of cells that were stored under refrigeration for 96 hours in UW+FP solution (control) or UW+FP solution with Def (UW+FP&Def solution) and then subjected to pre-warming treatment or not subjected to pre-warming treatment (control).

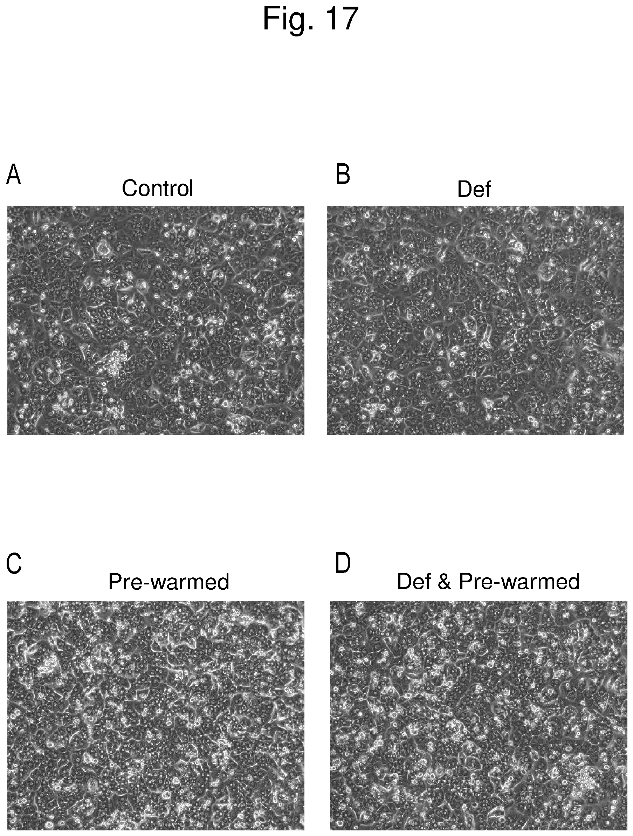

[Figure 17] Figure 17 shows phase-contrast image photos taken 24 hours after seeding cells onto plates, which had been stored under refrigeration for 96 hours in either UW+FP solution (control) or UW+FP solution with Def (UW+FP&Def solution) and then either subjected or not subjected to pre-warming treatment.

### Description of Embodiments

The present invention will now be described in detail.

The present invention provides a cell preservation solution comprising a high-molecular-weight polymer. The present invention also provides a method of preserving adherent cells, comprising storing cells, in particular, adherent cells, in a non-frozen state in the presence of a high-molecular-weight polymer, more specifically, in a cell preservation solution comprising a high-molecular-weight polymer. Furthermore, the present invention provides a method of culturing adherent cells, comprising culturing adherent cells that had been preserved according to such preservation method, in two-dimensional culture.

In particular, the present invention relates to a method of preserving adherent cells, comprising storing adherent cells in a non-frozen state under refrigeration in a cell preservation solution comprising a high-molecular-weight polymer.

In the present invention, the term "cell preservation solution" refers to a liquid composition suitable for stably storing cells while maintaining their viability. The cell preservation solution of the present invention is not the same as a liquid medium suitable for cell growth. In a preferred embodiment, the "cell preservation solution" of the present invention may be a cell preservation solution for non-cryopreservation or for storage under refrigeration. The "cell preservation solution" of the present invention is not a cell preservation solution for cryopreservation. The "cell preservation solution" of the present invention comprises an aqueous solvent and a high-molecular-weight polymer.

The "cell preservation solution" of the present invention may be prepared based on a commercially available or existing cell or organ preservation solution, or may be formulated de novo. In one embodiment, the "cell preservation solution" of the present invention may be prepared using non-cryopreservative Belzer UW^{(R)} Cold Storage Solution (UW Solution) as a base. The composition of Belzer UW^{(R)} Cold Storage Solution (UW solution) is as follows: hydroxyethyl starch (pentafraction) 50 g/L, lactobionic acid (as lactone) 35.83 g/L, potassium dihydrogen phosphate 3.4 g/L, magnesium sulfate heptahydrate 1.23 g/L, raffinose pentahydrate 17.83 g/L, adenosine 1.34 g/L, allopurinol 0.136 g/L, total glutathione 0.922 g/L, potassium hydroxide 5.61 g/L, sodium hydroxide/hydrochloric acid (added to adjust the pH to 7.4 at 20°C), and water. In one embodiment, a solution having or comprising the composition of Belzer UW^{(R)} Cold Storage Solution (UW solution) and comprising a high-molecular-weight polymer can be used as the cell preservation solution in the present invention.

In the present invention, the "high-molecular-weight polymer" refers to a polymer with a weight-average molecular weight of 10,000 or more. The molecular weight of a high-molecular-weight polymer can be determined, for example, by gel permeation chromatography (GPC) in terms of pullulan standards. High-molecular-weight polymers used in the present invention include, but are not limited to, polysaccharides, for example. In a preferred embodiment, the polysaccharide used as the high-molecular-weight polymer of the present invention may be an acidic polysaccharide having an anionic functional group. Specific examples of high-molecular-weight polymers include, but are not limited to, those composed of one or two or more selected from the group consisting of deacylated gellan gum, gellan gum, hyaluronic acid, rhamsan gum, diutan gum, xanthan gum, carrageenan, xanthan gum, hexuronic acid, fucoidan, pectin, pectic acid, pectinic acid, alginic acid, heparan sulfate, heparin, heparitine sulfate, keratosulfate, chondroitin sulfate, dermatan sulfate, and rhamnan sulfate, and salts thereof. Regarding high-molecular-weight polymers, the "salt" may be, for example, but is not limited to, salts of alkali metals such as lithium, sodium, and potassium; salts of alkaline earth metals such as calcium, barium, and magnesium; and salts of aluminum, zinc, copper, and iron, or the like; and ammonium salts, salts with organic amines, and salts with amino acids. In a more preferred embodiment, the high-molecular-weight polymers of the present invention comprise deacylated gellan gum or salt thereof. In one embodiment, the cell preservation solution of the present invention comprises only deacylated gellan gum or salt thereof as the high-molecular-weight polymer. In another embodiment, the cell preservation solution of the present invention comprises, in addition to deacylated gellan gum or salt thereof, other high-molecular-weight polymers, for example, alginic acid, pectin, or pectic acid, or other polysaccharides, as the high-molecular-weight polymers. In another embodiment, the cell preservation solution of the present invention comprises, as the high-molecular-weight polymers, in addition to deacylated gellan gum or salt thereof, an acidic polysaccharide (for example, alginic acid, pectin, or pectic acid) or salt thereof that maintains a random-coil state in a divalent metal cationic medium and can be crosslinked via divalent metal ions. When the cell preservation solution of the present invention comprises, as the high-molecular-weight polymers, in addition to deacylated gellan gum or salt thereof, an acidic polysaccharide (for example, alginic acid, pectin, or pectic acid) or salt thereof that maintains a random-coil state in a divalent metal cationic medium and can be crosslinked via divalent metal ions, the cell preservation solution may further comprise divalent metal cations such as calcium ion. Phosphorylated deacylated gellan gum or salt thereof may also be used. The weight-average molecular weight of the deacylated gellan gum or salt thereof may be preferably 10,000 to 50,000,000, more preferably 1,000,000 to 10,000,000. Commercially available deacylated gellan gum or salt thereof may also be used. In one embodiment, the FP series polymers manufactured by Nissan Chemical Corporation, Ltd., such as FP001, may be used. In one embodiment, FP001 solution, which is included as a component of the FCeM^{(R)} Preparation Kit (Nissan Chemical, product code 385-07981), can be used as the high-molecular-weight polymer reagent. In another embodiment, "KELCOGEL (registered trademark of CP Kelco) CG-LA" manufactured by Sansho Co., Ltd. may be used as the deacylated gellan gum or salt thereof.

In one embodiment, a cell preservation solution comprising the high-molecular-weight polymer of the present invention may comprise high-molecular-weight polymer(s), for example, deacylated gellan gum or salt thereof, at a concentration of 0.001 to 1% (w/v), preferably 0.005 to 0.3% (w/v), more preferably 0.01 to 0.3% (w/v), for example, 0.01 to 0.03% (w/v), or 0.015 to 0.03% (w/v). The concentration of deacylated gellan gum or salt thereof can be expressed in terms of the free-form deacylated gellan gum equivalent. In the present specification, % (w/v) means weight/volume %.

In the present invention, "adherent cells" refers to cells that can be cultured in maintenance culture in a state in which they are adherent (attached) onto a culture substrate. On the other hand, "suspension cells" refers to cells that are not adherent (attached) onto a culture substrate. Suspension cells gradually lose such function during culture in a liquid medium, making it difficult to perform maintenance culture over a long period of time. The "maintenance culture" refers to culturing cells while maintaining their morphology and function. Adherent cells can be successfully used in two-dimensional culture and three-dimensional culture.

The adherent cells used in the present invention may be derived from any organ or tissue, and include, for example, hepatocytes (i.e., adherent hepatocytes), but are not limited thereto. The adherent cells may be any animal cells, for example, mammalian cells such as primate cells, canine cells, or feline cells. The adherent cells may be human cells, or non-human cells, for example, non-human animal cells including non-human mammalian cells (such as non-human primate cells). In a preferred embodiment, the adherent cells may be human hepatocytes (i.e., human adherent hepatocytes).

The adherent cells used in the present invention are preferably, but are not limited to, primary cells. The adherent cells used in the present invention may or may not be immortalized cells. The adherent cells used in the present invention may be cells induced to differentiate from stem cells (e.g., iPS cells, mesenchymal stem cells). In the present invention, "primary cells" refers to cells which have been collected or isolated from biological tissue and are capable of proliferating (dividing) only a finite number of times. In the present invention, the "primary cells" refers not only to cells collected or isolated from naturally occurring biological tissue, but also to cells isolated from tissues or organs reconstructed by transplanting primary cells into the bodies of non-human animals (e.g., into the liver after induction of liver damage) having a deficient or reduced immune response to humans, such as immunodeficient or immunotolerant animals. Non-human animals having a deficient or reduced immune response to humans, which are used for reconstructing tissues or organs from primary cells, include, but are not limited to, non-human vertebrates having a deficient or reduced immune response to humans described in the specification and the like of International Publication WO2020/122178, for example, TK-NOG-hIL6 mice (hyper-immunodeficient mice in which a human thymidine kinase gene and a human IL-6 gene have been introduced in an expressible manner into NOG mice (non-human animal) derived from NOD/SCID mice and in which the IL2 receptor γ-chain gene has been knocked out).

The concentration of adherent cells in the cell preservation solution comprising the high-molecular-weight polymer during storage may be any cell concentration, but in one embodiment, the concentration may be preferably 1 x 10⁶ to 1 x 10⁸ cells/mL, more preferably 5 x 10⁶ to 7 x 10⁷ cells/mL, for example, 1 x 10⁷ cells/mL.

In the method of the present invention, adherent cells are stored in a cell preservation solution comprising the above-mentioned high-molecular-weight polymer, preferably in a non-frozen state, under refrigeration. In the present invention, "storage under refrigeration" means storage at a temperature condition of more than 0°C and no more than 10°C, typically 2 to 5°C, for example 4°C.

In the present invention, adherent cells can be stored for a long period of time in a cell preservation solution comprising a high-molecular-weight polymer in a non-frozen state (for example, stored under refrigeration). The time for which adherent cells are stored in a non-frozen state in a cell preservation solution comprising a high-molecular-weight polymer according to the present invention may be, but is not limited to, up to 100 hours, for example, up to 96 hours, or up to 72 hours. According to the present invention, the time for which adherent cells are stored in a non-frozen state in a cell preservation solution comprising a high-molecular-weight polymer may be 1 hour or more, typically 24 hours or more, preferably 40 hours or more, for example, 48 hours or more, 70 hours or more, or 72 hours or more, or alternatively, 1 to 100 hours, 24 to 100 hours, 40 to 100 hours, 48 to 100 hours, 70 to 100 hours, 72 to 100 hours, 1 to 96 hours, 24 to 96 hours, 40 to 96 hours, 48 to 96 hours, 70 to 96 hours, 72 to 96 hours, 72 to 100 hours, or 24 to 72 hours.

In the cell preservation solution of the present invention, adherent cells may be preferably in suspension,; in this case, adherent cells may be stored under refrigeration in suspension. In the present invention, maintaining cells in suspension in the preservation solution without settling is thought to reduce ischemic stress and thereby enhance preservation effect.

In the method of the present invention, adherent cells are stored in a non-frozen state (for example, stored under refrigeration) in a cell preservation solution comprising the above-mentioned high-molecular-weight polymer, thereby enabling stable storage (preservation) of the cells without impairing their adhesion capacity onto a culture substrate.

In the present invention, the "adhesion capacity" of adherent cells onto a culture substrate refers to the ability of adherent cells to attach onto a culture substrate, reach a confluent state, and maintain that confluent state when the adherent cells are seeded on the culture substrate and cultured in two-dimensional culture. In the present invention, the term "confluent state" refers to a state in which the seeded adherent cells occupy 80% or more of the surface area for culturing of the culture substrate (80% or more confluency). In the present invention, the term "adherent" refers to cells attaching to a culture substrate in a culturable state.

In a preferred embodiment, adherent cells stored under refrigeration in a non-frozen state in a cell preservation solution comprising the high-molecular-weight polymer maintain their adhesion capacity onto a culture substrate, and therefore can be suitably used for solid-phase culture on a culture substrate, in particular, two-dimensional culture (also called monolayer culture), after the storage under refrigeration in the cell preservation solution. Therefore, the preservation method of adherent cells of the present invention may be a method of storing adherent cells under refrigeration in a non-frozen state in a cell preservation solution comprising the high-molecular-weight polymer while maintaining their adhesion capacity onto a culture substrate in two-dimensional culture. Alternatively, the preservation method of adherent cells of the present invention may be a method for preserving adherent cells to be subjected to two-dimensional culture (for example, planar culture) after storage under refrigeration in a non-frozen state in a cell preservation solution comprising the high-molecular-weight polymer.

When primary hepatocytes (PHH) are suspended in a conventional cell preservation solution and stored under refrigerated conditions (4°C, on ice), they can be stored for a short period of time while maintaining their high adhesion capacity onto culture substrates. However, in that case, if they are stored under refrigeration for a long period of time, the adhesion capacity of PHH onto culture substrates decreases, and the cells become non-confluent, with gaps between the cells. PHH cultured in a non-confluent state with gaps between the cells changes cell morphology and loses hepatocyte properties (drug-metabolizing enzyme activity or the like). According to the method of the present invention, adherent cells, including hepatocytes, can maintain their adhesion capacity onto culture substrates by storing them under refrigeration in a non-frozen state in a cell preservation solution comprising the high-molecular-weight polymer, and, as a result, also maintain other cellular properties of the adherent cells. In a preferred embodiment, adherent cells stored under refrigeration in a cell preservation solution comprising the high-molecular-weight polymer maintain their cell morphology. In a preferred embodiment, adherent cells stored under refrigeration in a cell preservation solution comprising the high-molecular-weight polymer maintain their cell-specific enzyme activities. For example, when adherent hepatocytes are stored under refrigeration as described above, their cell morphology and drug-metabolizing enzyme activities are maintained. Drug-metabolizing enzyme activities include, but are not limited to, for example, cytochrome P450 enzyme activities such as CYP1A2-mediated phenacetin O-deethylation activity, CYP2C9-mediated diclofenac 4'-hydroxylation activity, CYP2C19-mediated omeprazole 5'-hydroxylation activity, CYP2D6-mediated metoprolol O-demethylation activity, and CYP3A4/5-mediated midazolam 1'-hydroxylation activity, and in the present specification. Furthermore, adherent cells stored under refrigeration in a cell preservation solution comprising the high-molecular-weight polymer remain in a healthier state, as indicated by, for example, increased intracellular ATP levels or the like, and exhibit suppression of increase of ROS activity, which is indicative of ischemic stress. Adherent cells stored under refrigeration in a cell preservation solution comprising the high-molecular-weight polymer exhibit an increased adhesion efficiency (plating index) onto culture substrates when seeded after storage under refrigeration. Storing adherent cells under refrigeration in a cell preservation solution comprising the high-molecular-weight polymer significantly reduces ischemic stress on the adherent cells, which is thought to contribute to the above-mentioned effects.

In the present invention, an iron chelator may be further added to the cell preservation solution comprising the high-molecular-weight polymer. In the present invention, a cell preservation solution comprising an iron chelator in addition to the high-molecular-weight polymer can be used. The iron chelator may comprise, but not limited to, deferoxamine and/or ferrostatin or the like. The iron chelator may be added to the cell storage solution containing the high molecular weight polymer at a concentration of, preferably but not limited to, 10 µM to 5 mM, for example, 100 µM to 1 mM, or 300 µM to 700 µM. By further adding an iron chelator to the cell preservation solution comprising the high-molecular-weight polymer, a further increase in the adhesion efficiency (plating index) of adherent cells is exhibited, and the adhesion capacity of adherent cells is improved.

Adherent cells stored under refrigeration according to the above-described preservation method of adherent cells maintain high adhesion capacity onto culture substrates and therefore can be used for two-dimensional culture. The present invention also provides a method for culturing adherent cells, comprising storing adherent cells under refrigeration in a cell preservation solution comprising the above-described high-molecular-weight polymer according to the above-described preservation method of adherent cells, and then subjecting the adherent cells to two-dimensional culture.

The method for culturing adherent cells may comprise storing adherent cells under refrigeration according to the preservation method of adherent cells, seeding the adherent cells on a culture substrate, and performing two-dimensional culture of the cells.

In the present invention, a culture substrate refers to a solid-phase material or substance to which cells adhere during culture and which functions as a scaffold. Examples of culture substrates include, but are not limited to, for example, a culture plate including a multiwell plate, a culture dish, a flask, a bottle, a glass slide, a cover glass, a film, a membrane, a porous carrier, a hollow fiber, a fiber, or the like. Culture substrates may be made of any cell scaffold material including, but not limited to, glass; plastics such as polystyrene, polyethylene terephthalate, polysulfone, polyethersulfone, and polycarbonate; metals such as silver and gold; metal oxides such as indium-tin oxide; and ceramic. The surface (culture surface) of the culture substrate may also be coated with scaffold material such as collagen, elastin, fibronectin, vitronectin, laminin, gelatin, or the like.

In one embodiment, adherent cells stored in a cell preservation solution comprising the high-molecular-weight polymer may be seeded on a collagen-coated culture substrate, followed by two-dimensional culture. Collagen used for the coating includes, but is not limited to, collagen I, collagen nanofiber, or the like.

Two-dimensional culture is a monolayer culture on a culture substrate (solid phase). When the surface of the culture substrate on which adherent cells are seeded is flat, the two-dimensional culture is called planar culture. Two-dimensional culture on a culture substrate of adherent cells stored under refrigeration in a cell preservation solution may be planar culture.

Two-dimensional culture of adherent cells can be performed under standard culture conditions for two-dimensional culture. Two-dimensional culture is preferably performed under warm conditions. In one embodiment, two-dimensional culture can be performed preferably at 30 to 40°C, more preferably at 35 to 40°C, even more preferably at 35 to 38°C, and typically at 37°C. In one embodiment, two-dimensional culture can be performed for, for example, 1 hour to 6 months, preferably 10 hours to 3 months, or 20 hours to 2 weeks.

In the method of the present invention, adherent cells stored under refrigeration in a cell preservation solution comprising the high-molecular-weight polymer may be warmed and then subjected to two-dimensional culture. The warming treatment may be any warming treatment that can reduce cold stress on cells. Warming treatment can be performed by suspending adherent cells stored under refrigeration in a cell preservation solution comprising the high-molecular-weight polymer in a liquid seeding medium, followed by incubation at preferably 30 to 40°C, more preferably 35 to 40°C, even more preferably 35 to 38°C, typically 37°C, and for 20 to 60 minutes, for example, 30 to 60 minutes. By warming adherent cells stored under refrigeration and then subjecting them to two-dimensional culture, cold stress in adherent cells stored under refrigeration can be reduced and the adhesion efficiency (plating index) can be improved.

The present invention also provides a cell preservation solution comprising the high-molecular-weight polymer as described above, which is suitable foruse in the cell preservation method and cell culture method of the present invention. The cell preservation solution comprising the high-molecular-weight polymer of the present invention is suitable for use in storing cells, particularly adherent cells, in a non-frozen state for a long period of time (for example, for storage under refrigeration). The cell preservation solution comprising the high-molecular-weight polymer of the present invention may be for use in storing adherent cells in a non-frozen state for up to 100 hours, for example, up to 96 hours, or up to 72 hours under refrigeration. The cell preservation solution comprising the high-molecular-weight polymer of the present invention may be for use in storing adherent cells in a non-frozen state under refrigeration for 1 hour or more, typically 24 hours or more, preferably 40 hours or more, for example, 48 hours or more, 70 hours or more, or 72 hours or more, or alternatively, 1 to 100 hours, 24 to 100 hours, 40 to 100 hours, 48 to 100 hours, 70 to 100 hours, 72 to 100 hours, 1 to 96 hours, 24 to 96 hours, 40 to 96 hours, 48 to 96 hours, 70 to 96 hours, 72 to 96 hours, 72 to 100 hours, or 24 to 72 hours.

The cell preservation solution comprising the high-molecular-weight polymer of the present invention may also be for use in storing adherent cells under refrigeration while maintaining their adhesion capacity onto a culture substrate in two-dimensional culture. The cell preservation solution comprising the high-molecular-weight polymer of the present invention may be for use in preserving adherent cells to be subjected to two-dimensional culture after being stored under refrigeration for a long period of time in a non-frozen state.

The cell preservation solution comprising the high-molecular-weight polymer of the present invention can be used to maintain the adhesion capacity of adherent cells onto a culture substrate during storage under refrigeration and to improve the preservation of adherent cells during storage under refrigeration. The cell preservation solution comprising the high-molecular-weight polymer of the present invention is particularly suitable for storing hepatocytes, including human hepatocytes, under refrigeration.

### Examples

Hereinafter, the present invention will be described in more detail with reference to Examples. However, the technical scope of the present invention is not limited to these Examples.

In the following Examples, all p-values in statistical analyses were calculated by paired t-tests of two groups, and p < 0.05 was regarded as indicating a significant difference.

### [Example 1] Preparation of a cell preservation solution containing a high-molecular-weight polymer and preservation of cells in the preservation solution

FCeM^{(R)} Preparation Kit (Nissan Chemical, product code 385-07981), which contains high-molecular-weight polymer FP001 solution as a component, was used for preparing a cell preservation solution. High-molecular-weight polymer FP001 solution contains deacylated gellan gum. Belzer UW^{(R)} cold preservation solution (Preservation Solutions, Inc.; purchased from Astellas Pharma; product code 01544782; hereinafter also referred to as the UW solution) was used as a base solution for the cell preservation solution.

Following the instructions provided with the FCeM^{(R)} Preparation Kit, FP001 solution was added to the UW solution and mixed to prepare a cell preservation solution (UW+FP solution; final concentration of high-molecular-weight polymer: 0.02% (w/v)). The prepared UW+FP solution was used without dilution for cell preservation as described below.

To evaluate the cell preservation effect achieved in the UW+FP solution, human hepatocyte HepaSH cells (Uehara et al., Biochemical and Biophysical Research Communications, 663 (2023) 132-141; Central Institute for Experimental Medicine and Life Science (CIEM), Japan) were used. HepaSH cells are cells isolated from humanized livers reconstructed by transplanting primary human hepatocytes (PHH) into the livers of hyperimmunodeficient TK-NOG-hIL6 mice (Uehara et al., Biochemical and Biophysical Research Communications, 663 (2023) 132-141; International Publication WO 2020/122178) after induction of liver damage. HepaSH cell from six specimens isolated from six different mice were used in this Example.

The HepaSH cells were added to the UW solution or the UW+FP solution to prepare cell suspensions at a concentration of 1 x 10⁷ cells/mL. The resulting cell suspensions were then stored at 4°C for 96 hours (stored under refrigeration) in 15 mL centrifuge tubes (Greiner Bio-One) as storage containers. Figure 1 shows the appearance of cells in the preservation solution 24 hours after the start of storage. Cells suspended in the UW solution settled, whereas cells suspended in the UW+FP solution remained floating without sedimentation even after 24 hours of storage.

Cells in the UW solution or UW+FP solution were sampled every 24 hours during the 96-hour storage period. To the obtained cell samples, trypan blue solution (Thermo Fisher Scientific, product code 15250061) was added, and the viable cells and total cells were counted using a hemocytometer (FMG, product code 521-10). The ratio of the viable cell count at each sampling time point to the viable cell count at the start of storage (viable cell recovery rate; % Recovery) and the ratio of the viable cell count to the total cell count (including viable and dead cells) at each sampling time point (viability rate; % Viability) were calculated. The results are shown in Figure 2. No apparent differences were observed in the viable cell recovery rate and viability rate between cells stored in the UW+FP solution and those stored in the UW solution, indicating that addition of the high-molecular-weight polymer FP does not affect these rates.

Further, the cells sampled from the UW solution or the UW+FP solution, which were obtained every 24 hours during the 96-hour storage period, were tested for their post-storage adhesion capacity onto scaffolds. Cell samples were suspended in a seeding medium (Williams' E medium + 10% fetal bovine serum, 100 U/mL penicillin, 100 mg/mL streptomycin, and 5 mg/mL insulin) at a concentration of 1 x 10⁶ cells/mL. The resulting cell suspension was seeded at 0.5 mL per well onto collagen I-coated 24-well plates (Corning; product code 356408) and cultured overnight in a CO₂ incubator (37°C, 5% CO₂). Twenty-four hours after seeding, the medium was replaced with the seeding medium containing the cell nuclear staining fluorescent dye Hoechst 33342 (Dojindo Laboratories, product code H342). After incubation for 30 minutes for cell staining, phase-contrast images were acquired using EVOS cell imaging systems (Thermo Fisher Scientific), and the average number of nuclei per unit area (within a 200-µm grid) was determined. For comparison, freshly isolated HepaSH cells from a humanized liver were, in the same manner as described above, suspended in the seeding medium, seeded onto collagen I-coated 24-well plates, cultured for 24 hours, and then stained, followed by determination of the average number of nuclei. The ratio of the average number of nuclei 24 hours after seeding of HepaSH cells stored in the cell preservation solution relative to the average number of nuclei 24 hours after seeding of freshly isolated HepaSH cells (100%) was calculated and used as the adhesion efficiency (plating index). The results are shown in Figures 3 and 4.

Cells stored in the UW+FP solution showed a higher adhesion efficiency than cells stored in the UW solution throughout the storage period, and even after 72 hours of storage, the adhesion efficiency was approximately 80% (Figure 3). It was observed from the phase-contrast images (Figure 4) that cells stored in the UW solution for 48 hours or longer exhibited gaps between cells during planar culture and failed to maintain a confluent state, whereas cells stored in the UW+FP solution were maintained in planar culture in a confluent state without gaps between cells even after 72 hours of storage.

These results show that storage of cells in a preservation solution containing a high-molecular-weight polymer enables the adhesion capacity of the cells to be maintained even after long-term storage.

### [Example 2] Reduction of ischemic stress in cells stored in a preservation solution containing a high-molecular-weight polymer

HepaSH cells (30 specimens) were suspended in the UW solution or the UW+FP solution at a concentration of 1 x 10⁷ cells/mL as in Example 1 and stored at 4°C for 24 hours. The number of viable cells after storage at 4°C for 24 hours was counted.

Then, for the cells stored at 4°C for 24 hours, the ATP level per 1,000 cells was determined using the CellTiter-Glo^{(R)} 2.0 Cell Viability Assay (Promega) and measuring luminescence with an EnSpire multimode plate reader (PerkinElmer). The results are shown in Figure 5A. Cells stored in the UW+FP solution showed statistically significantly higher levels of ATP than those stored in the UW solution. During cell death, intracellular ATP levels decrease. Therefore, these results show that storing cells in the presence of high-molecular-weight polymers maintains the cells in a healthier state, even after prolonged storage.

Furthermore, for the cells stored at 4°C for 24 hours, the amount of reactive oxygen species (ROS; ROS activity), which is an indicator of ischemic stress in cells after storage, was measured using a total ROS detection kit (Dojindo Laboratories, product code R252). The cells stored at 4°C for 24 hours were stained with an anti-HLA antibody (BD, product code 555555) and the Highly Sensitive DCFH-DA dye included in the above kit according to the kit's instructions, and then suspended in a loading buffer (1x) supplemented with propidium iodide (PI). The stained cells were analyzed using a FACS Aria II cell sorter (BD). The mean fluorescence intensity (FITC mean) derived from DCFH-DA was calculated in a cell polulation that was PI-negative (indicating viable cells) and HLA-positive (indicating human hepatocytes), and the calculated values were compared between the UW solution and the UW+FP solution. The results are shown in Figure 5B. The FITC mean of the cells stored in the UW solution was statistically significantly higher compared with that of cells stored in the UW+FP solution. In this assay, the greater the amount of intracellular ROS accumulation, the higher the FITC mean. Thus, cells stored in the UW solution exhibited increased ROS accumulation, indicating enhanced ischemic stress. These results show that storage in the presence of high-molecular-weight polymers reduces ischemic stress in cells.

Furthermore, the cells stored at 4°C for 24 hours were suspended in the above-mentioned seeding medium, seeded onto collagen I-coated 24-well plates, and cultured for 4 hours. The culture supernatant was then collected. Lactate dehydrogenase (LDH) activity in the collected culture supernatant was measured as an indicator of cell death, using the CytoTox-ONE Homogeneous Membrane Integrity Assay (Promega, product code G7890) according to the instructions. Fluorescence measurements were performed using an Infinite F200F Pro plate reader (TECAN). The fluorescence intensity measured for the culture supernatant was normalized to that measured in unused seeding medium serving a control medium. The results are shown in Figure 5C. Cells stored in the UW solution had statistically significantly higher LDH activity than cells stored in the UW+FP solution. When cell death increases, LDH leaks from cells into the medium, increasing LDH activity in the culture supernatant. This indicates that storage in the UW solution increased cell death after seeding. It is believed that storage in the UW solution increased cell death after seeding due to increased ischemic stress. These results show that storage in the presence of high-molecular-weight polymers enables reduction of cell death due to increased ischemic stress after seeding onto a culture substrate.

### [Example 3] Maintenance of cell characteristics after storage in cell preservation solution

HepaSH cells (four specimens) were suspended in the UW+FP solution at a concentration of 1 x 10⁷ cells/mL as in Example 1 and stored at 4°C for 72 hours. The cells thus stored under refrigeration for 72 hours were seeded onto collagen I-coated 24-well plates and cultured for 8 days (maintenance culture). As a control, freshly isolated HepaSH cells from a humanized liver were seeded onto collagen I-coated 24-well plates and similarly cultured for 8 days (maintenance culture). Four hours after seeding, the medium was changed to Cellartis^{(R)} Power^{™} Primary HEP medium (Takara Bio, product code Y20020), and then the medium was replaced with the same medium on Days 1 (the day after seeding), 3, 5, and 7 of culture. Figure 6 shows phase-contrast images of cells on Days 1, 3, 5, and 7 of culture. Throughout the culture period up to Day 7, no difference in cell morphology was observed regardless of whether the cells had been stored in a cell preservation solution, and the cells maintained a confluent state in planar culture.

Furthermore, during the culture period up to Day 7, the activities of major drug-metabolizing enzymes in the cells were measured on Days 1, 4, and 8 of culture. HepaSH cells harvested on Days 1, 4, and 8 of culture were incubated at 37°C for 1 hour in Williams' Medium E, no Phenol Red (Thermo Fisher Scientific, product code A12176) supplemented with phenacetin (a substrate for CYP1A2) (100 µM), diclofenac (a substrate for CYP2C9) (40 µM), omeprazole (a substrate for CYP2C19) (10 µM), metoprolol (a substrate for CYP2D6) (5 µM), and midazolam (a substrate for CYP3A4/5) (5 µM), which are typical substrates of human drug-metabolizing cytochrome P450 enzymes. Metabolites contained in the medium were then measured using a liquid chromatography-tandem mass spectrometer. The following metabolites, which were derived from the substrates, were measured as indicators of the corresponding drug-metabolizing enzyme activities: acetaminophen, which is produced by O-deethylation of phenacetin by CYP1A2; 4'-hydroxydiclofenac, which is produced by 4'-hydroxylation of diclofenac by CYP2C9; 5'-hydroxyomeprazole, which is produced by 5'-hydroxylation of omeprazole by CYP2C19; O-demethylated metoprolol, which is produced by O-demethylation of metoprolol by CYP2D6; and 1'-hydroxymidazolam, which is produced by 1'-hydroxylation of midazolam by CYP3A4/5. Furthermore, HepaSH cells in each well were lysed in CelLytic^{™} MT Cell Lysis Reagent (Sigma-Aldrich, product code C3228), and protein concentration was quantified using the TaKaRa BCA Protein Assay Kit (Takara Bio, product code T9300A). The drug-metabolizing enzyme activities measured as described above were expressed as the amount of metabolite produced per unit time and per unit protein weight. Figure 7 shows the drug-metabolizing enzyme activities expressed as the amount of metabolite produced per unit protein weight. Compared with the drug-metabolizing enzyme activities in cells not stored under refrigeration, HepaSH cells stored under refrigeration for 72 hours in the UW+FP solution maintained drug-metabolizing enzyme activities throughout the culture period until Day 8.

These results indicate that cells stored in the presence of high-molecular-weight polymers maintain their cellular properties even after prolonged storage under refrigeration. In particular, the ability of hepatocytes to maintain their drug-metabolizing enzyme activities is crucial for storage, transport, etc. of hepatocytes.

Furthermore, HepaSH cells, which were suspended in the UW+FP solution at a concentration of 1 x 10⁷ cells/mL and stored at 4°C for 72 hours, were transplanted via the spleen into the livers of TK-NOG-hIL6 mice after induction of liver damage. Five weeks after transplantation, the liver was harvested and fixed in formalin. Paraffin sections were prepared, stained with hematoxylin and eosin (H&E) and immunostained with an anti-human mitochondria antibody (Merck, product code M AB1273). Images were taken using a NanoZoomer slide scanner (Hamamatsu Photonics). The results are shown in Figure 8. It was shown that the majority of the recipient mouse liver was replaced with human hepatocytes that were positive for human mitochondria (Figures 8A and B). In addition, HepaSH cells stored under refrigeration for 72 hours were transplanted into the liver of a separate TK-NOG-hIL6 mouse after induction of liver damage. Six weeks after transplantation, HepaSH cells were re-isolated from this mouse. The isolated cells were stained with an anti-HLA antibody and an anti-H2kD antibody (BD, product code 553566) and analyzed by flow cytometry. No less than 96% of the isolated cells were HLA-positive human hepatocytes (Figure 9A). The re-isolated HepaSH cells were seeded on collagen I-coated plates, and successfully established an adherent culture as observed with the cells before transplantation (Figure 9B). These results indicate that hepatocytes stored in the presence of high-molecular-weight polymers maintain their liver reconstitution capacity even after prolonged storage under refrigeration.

### [Example 4] Effects of adding iron chelator and pre-warming

### 1) Addition of iron chelator

We hypothesized that an iron chelator, which removes free iron ions from cells, could prevent ferroptosis, an iron-dependent cell death. Therefore, we examined whether the addition of an iron chelator to a cell preservation solution, in combination with the addition of a high-molecular-weight polymer, would further improve the adhesion capacity of cells stored in the cell preservation solution.

HepaSH cells (9 specimens) were suspended at a concentration of 1 x 10⁷ cells/mL in the UW solution or the UW+FP solution, with or without an iron chelator (final concentration of 500 µM, deferoxamine; hereinafter also referred to as Def), and stored at 4°C for 24 hours or 96 hours (storage under refrigeration). Hereinafter, the UW solution with Def is referred to as the UW+Def solution, and the UW+FP solution with Def is referred to as the UW+FP&Def solution.

The cells after storage under refrigeration were seeded onto collagen I-coated 24-well plates. 24 hours after seeding, the cells were stained and the average number of nuclei was measured. The adhesion efficiency (plating index) was calculated from the measured average number of nuclei in the same manner as in Example 1.

The results are shown in Figures 10 and 11. After 24 hours of storage under refrigeration, cells stored in the UW+FP solution, UW+Def solution, and UW+FP&Def solution exhibited a statistically significant increase in adhesion efficiency compared to the UW solution (non-supplemented), but no additive effect was observed with the combination of high-molecular-weight polymer and Def (Figure 10). On the other hand, after 96 hours of storage under refrigeration, cells stored in the UW+FP solution, UW+Def solution, and UW+FP&Def solution exhibited a statistically significant increase in adhesion efficiency compared to the UW solution (non-supplemented). Furthermore, the combination of high molecular weight polymer and Def in the UW+FP&Def solution exhibited a statistically significant increase in adhesion efficiency compared to the addition of high-molecular-weight polymer or Def alone (Figure 10). In addition, in particular, cells stored under refrigeration for 96 hours failed to maintain a confluent state in the subsequent planar culture when stored in the UW solution or UW \+Def solution, whereas phase-contrast images also showed that cells maintained a confluent state in planar culture when stored in the UW+FP solution or UW+FP&Def solution (Figure 11). It was shown that the combined use of a high-molecular-weight polymer and an iron chelator, which prevents both ischemic stress and ferroptosis, results in maintenance of the cellular adhesion capacity required for confluent culture even after 96 hours of storage in a cell preservation solution.

Also, HepaSH cells were suspended in the UW solution, UW+FP solution, or UW+FP&Def solution at a concentration of 1 x 10⁷ cells/mL. After storage at 4°C for 96 hours, the cells were suspended in seeding medium and seeded onto Transwell plates (Corning, product code 354495) coated with rat collagen I (Corning, product code 354236). The cells were subjected to a 7-day maintenance culture as planar culture. On Day 7 of culture, medium supplemented with PE streptavidin (BD, product code 554061) was added to the upper chamber of the Transwell. The medium in the lower chamber was collected 15, 30, and 60 minutes after the addition of PE streptavidin, and fluorescence was measured using an Infinite F200F Pro plate reader (TECAN). In this assay, if the confluent state is not maintained in planar culture, PE-streptavidin is transferred from the upper chamber to the lower chamber through intercellular gaps, and fluorescence derived from the fluorescent dye PE (phycoerythrin) is detected in the medium.

For comparison, fluorescence measurements were similarly performed on wells seeded with freshly isolated HepaSH cells from humanized liver (Fr) and on wells not seeded with cells (No Cells). The measured fluorescence intensities were normalized to that measured in unused seeding medium (control medium). The measurement results of the fluorescence intensities are shown in Figure 12. Figure 13 shows phase-contrast images of cells on Day 7 of planar culture.

In cells stored in the UW solution for 96 hours, intercellular gaps were observed in phase-contrast images (Figure 13B), and fluorescence was detected 15 minutes after the addition (cell exposure) of PE-streptavidin (Figure 12). In contrast, in cells stored in the UW+FP solution or UW+FP&Def solution for 96 hours, intercellular gaps were not observed in phase-contrast images (Figures 13C and 13D). Similar to the freshly isolated HepaSH cells (Fr), almost no fluorescence was detected even 30 minutes after the addition of PE-streptavidin (Figure 12). These results demonstrate that HepaSH cells stored under refrigeration for 96 hours in the presence of a high-molecular-weight polymer or a combinartion of a high-molecular-weight polymer and an iron chelator maintained a confluent state even after 7 days of monolayer culture.

### 2) Alleviation of cold stress by pre-warming

We hypothesized that cell death after seeding could be suppressed by suspending cells in seeding medium after cold storage, incubating them at 37°C for 30-60 minutes, and then seeding them on collagen I-coated plates. Therefore, we examined the effect of the combined use of adding a high-molecular-weight polymer to the cell preservation solution and warming it before seeding (pre-warming).

HepaSH cells (10 specimens) were suspended in the UW solution or UW+FP solution at a concentration of 1 x 10⁷ cells/mL as in Example 1 and stored at 4°C for 72 hours. Half of the suspension was then collected and suspended in 10 times the volume of seeding medium. The cells were then pre-warmed by incubating at 37°C for 30 minutes in a thermostatic water bath.

For the cells subjected to pre-warming treatment, the viable cell recovery rate and viability rate were calculated by staining with trypan blue and counting the viable and total cell numbers using a hemocytometer according to the method described in Example 1. Furthermore, according to the method described in Example 1, phase-contrast images were taken 24 hours after seeding, and the adhesion efficiency (plating index) was determined. Furthermore, ROS activity was measured according to the method described in Example 2. As a control, a similar storage test and measurement were performed except that the pre-warming treatment was not performed. The results are shown in Figures 14 and 15.

Pre-warming treatment tended to decrease the viable cell recovery rate in cells stored in the UW solution, whereas the viable cell recovery rate remained largely unchanged in cells stored in the UW+FP solution (Figure 15A). Pre-warming promotes the death of damaged cells with accumulated ischemic stress. Therefore, it is considered that the viable cell recovery rate decreased in cells stored in the UW solution due to accumulated ischemic stress, whereas the viable cell recovery rate did not decrease in cells stored in the UW+FP solution due to less accumulated ischemic stress and fewer damaged cells (Figure 15A).

Meanwhile, it was shown that pre-warming treatment resulted in a statistically significant improvement in the viability rate of cells stored in either the UW solution or UW+FP solution (Figure 15B). Furthermore, it was shown that pre-warming treatment resulted in a statistically significant reduction in ROS activity and increase in adhesion efficiency for cells stored in either the UW solution or UW+FP solution (Figures 15C and 15D). Pre-warming also has the effect of reducing cellular cold stress, which is thought to have resulted in the improvement in viability rate, reduction in ROS activity, and increase in adhesion efficiency.

These results indicate that pre-warming treatment alleviates the cold stress in cells after storage under refrigeration, even in the presence of high-molecular-weight polymers.

### 3) Combined use of iron chelator addition and pre-warming for alleviating cold stress

HepaSH cells (6 specimens) were suspended in the UW+FP solution or UW+FP&Def solution at a concentration of 1 x 10⁷ cells/mL. After storage at 4°C for 96 hours, the cells were suspended in 10 volumes of seeding medium and pre-warmed by incubating the cells at 37°C for 30 minutes using a thermostatic water bath. The pre-warmed cells were seeded onto collagen I-coated 24-well plates and cultured overnight. Phase-contrast images were then taken 24 hours after seeding, and the adhesion efficiency (plating index) was determined according to the method described in Example 1. The results are shown in Figures 16 and 17. The addition of iron chelator and the pre-warming treatment each showed a statistically significant increase in the adhesion efficiency. The combination of an iron chelator and a pre-warming treatment resulted in a statistically significant increase in the adhesion efficiency compared to the control (the UW+FP solution, no iron chelator and no pre-warming treatment). However, no additive or synergistic effects were observed with the combination of an iron chelator and a pre-warming treatment. The phase-contrast images showed that cells stored under all refrigeration storage conditions maintained a confluent state in planar culture (Figure 17).

Examples 1 to 4 demonstrate that ischemic stress in cells during storage in cell preservation solutions becomes apparent after the cells after storage are seeded on a culture substrate and culture is initiated. It was shown by Examples 1 to 4 that cells survived despite ischemic stress during storage in cell preservation solutions undergo increased cell death when seeded on a culture substrate after storage and cultured, resulting in a decrease in the adhesion efficiency of the cells in culture on the culture substrate. Furthermore, it was shown that the present invention significantly reduces such cell death and decrease in adhesion efficiency.

### Industrial Applicability

The present invention maintains the adhesion capacity of adherent cells onto a culture substrate when stored under refrigeration for a long period of time in a non-frozen state, thereby enabling long-distance transport such as air transport of adherent cells to be subjected to two-dimensional culture after storage under refrigeration.

All publications, patents, and patent applications cited in the description are incorporated in their entirety by reference.

## Claims

1. A method of preserving adherent cells, comprising storing adherent cells in a non-frozen state under refrigeration in a cell preservation solution comprising a high-molecular-weight polymer.

2. The method according to claim 1, wherein the high-molecular-weight polymer comprises deacylated gellan gum or a salt thereof.

3. The method according to claim 1, wherein the adherent cells are stored under refrigeration while maintaining their adhesion capacity onto a culture substrate in two-dimensional culture.

4. The method according to claim 1, wherein the adherent cells are stored under refrigeration for up to 100 hours.

5. The method according to claim 1, wherein the adherent cells are stored under refrigeration for 70 hours or more.

6. The method according to claim 1, wherein the adherent cells are hepatocytes.

7. The method according to claim 1, wherein the adherent cells are stored in suspension in a cell preservation solution.

8. The method according to claim 1, wherein the cell preservation solution further comprises an iron chelator.

9. The method according to claim 1, wherein the iron chelator comprises deferoxamine.

10. A method of culturing adherent cells, comprising storing adherent cells under refrigeration using the method according to any one of claims 1 to 9, and then culturing the adherent cells in two-dimensional culture.

11. The method according to claim 10, wherein the adherent cells are seeded on a collagen-coated culture substrate and cultured in two-dimensional culture.

12. The method according to claim 10, wherein the adherent cells that have been stored under refrigeration are warmed and then subjected to two-dimensional culture.

13. A cell preservation solution comprising a high-molecular-weight polymer, for preserving adherent cells in a non-frozen state under refrigeration.

14. The cell preservation solution according to claim 13, wherein the high-molecular-weight polymer comprises deacylated gellan gum or a salt thereof.

15. The cell preservation solution according to claim 13, for preserving adherent cells under refrigeration while maintaining their adhesion capacity onto a culture substrate in two-dimensional culture.

16. The cell preservation solution according to claim 13, for preserving adherent cells under refrigeration for up to 100 hours.

17. The cell preservation solution according to claim 13, wherein the adherent cells are hepatocytes.

18. The cell preservation solution according to claim 13, which further comprises an iron chelator.
